Europäisches Patentamt

European Patent Office

Office européen des brevets

(11) Numéro de publication : **0 128 058
B1**

(12) **FASCICULE DE BREVET EUROPÉEN**

(45) Date de publication du fascicule du brevet :
**13.04.88**

(51) Int. Cl.⁴ : **A 61 F   5/02**

(21) Numéro de dépôt : **84400841.7**

(22) Date de dépôt : **25.04.84**

(54) **Dispositif pour l'étaiement du rachis.**

(30) Priorité : **04.05.83 FR 8307450**

(43) Date de publication de la demande :
**12.12.84 Bulletin 84/50**

(45) Mention de la délivrance du brevet :
**13.04.88 Bulletin 88/15**

(84) Etats contractants désignés :
**BE CH DE GB IT LI LU NL**

(56) Documents cités :
**DE-A- 3 032 237
DE-B- 2 649 042
FR-A- 2 405 063
FR-A- 2 458 271**

(73) Titulaire : **SOCIETE DE FABRICATION DE MATERIEL
ORTHOPEDIQUE SOFAMOR Société à responsabilité
limitée dite:
60,62 rue Rothschild
F-62100 Berck-Plage (FR)**

(72) Inventeur : **Cotrel, Yves Paul Charles Alexandre
Villa Kerosen Taden
F-22100 Dinan (FR)**

(74) Mandataire : **Bonnetat, Christian et al
Cabinet PROPI Conseils 23 rue de Léningrad
F-75008 Paris (FR)**

Jouve, 18, rue St-Denis, 75001 Paris, France

## Description

La présente invention concerne un dispositif pour l'étaiement du rachis, mis en place par intervention opératoire. Le dispositif selon l'invention peut être utilisé pour simplement étayer un rachis qui en a besoin (fracture accidentelle, par exemple) ou bien encore pour redresser et étayer un rachis objet de déviations (scolioses, cyphoses par exemple) · Quoique le dispositif soit apte à ces deux types d'application, il sera plus spécialement décrit ci-après en rapport avec le traitement chirurgical des déviations latérales de la colonne vertébrale.

On sait que la scoliose est la déviation latérale de la colonne vertébrale, accompagnée de la rotation de certaines vertèbres sur elles-mêmes. La courbure rachidienne anormale qui résulte de cette déviation latérale est généralement définie à partir de repères précis, à savoir les vertèbres extrêmes supérieure et inférieure entre lesquelles a lieu ladite courbure anormale et la ou les vertèbres du sommet de celle-ci. Les vertèbres extrêmes supérieure et inférieure sont celles qui sont le moins tournées autour de leur axe, mais en revanche elles sont les plus inclinées sur l'axe longitudinal médian et leurs plans déterminent entre eux l'angle de courbure scoliotique. La ou les vertèbres du sommet sont celles qui sont les plus éloignées de cet axe médian du tronc : elles sont peu ou pas inclinées par rapport à ce dernier axe, mais en revanche elles subissent la plus forte rotation sur elles-mêmes.

Lorsque l'angle de la courbure scoliotique dépasse un certain seuil (voisin de 50°), il devient nécessaire d'envisager le traitement chirurgical de la scoliose, appelé arthrodèse et consistant à souder ensemble toutes les vertèbres de la courbure scoliotique, après correction maximale de celle-ci par redressement et ouverture.

Une telle correction peut être préparée antérieurement à l'opération par traction continue du rachis ou usage de plâtres correcteurs. Toutefois, c'est au cours du stade opératoire que cette correction est parachevée et rendue définitive. A cet effet, au moins dans la concavité de la courbure rachidienne, on place un étai solide, qui fixe le redressement obtenu et qui est susceptible d'armer le bloc osseux réalisé par l'arthrodèse.

Pour ce faire, on connaît déjà l'instrumentation dite de « HARRINGTON ». Cette instrumentation comporte un système d'élongation destiné à être inséré dans la concavité de la courbure et un système de compression destiné à être inséré éventuellement du côté de la convexité. En fait, très souvent, seul le système d'élongation est utilisé.

Ce système d'élongation comprend deux organes d'ancrage métalliques, du type crochet, prenant appui sur les vertèbres extrêmes de la courbure, et une tige métallique, faisant office d'étai, permettant d'obtenir et de maintenir l'écartement des crochets, l'un par rapport à l'autre, et donc le redressement de la courbure scoliotique.

Cette tige métallique, de section circulaire, comporte un épaulement à sa partie inférieure et une portion crantée à sa partie supérieure et elle traverse lesdits crochets. Le crochet inférieur est accroché sur la lame de la vertèbre extrême inférieure de la courbure et la tige métallique prend appui sur ledit crochet inférieur par l'intermédiaire dudit épaulement. Le crochet supérieur, enfilé sur ladite tige, est disposé sous l'apophyse articulaire de la vertèbre extrême supérieure de la courbure et par des manœuvres externes de traction (par exemple au moyen d'une pince écartante prenant appui sur les crans de la partie supérieure de la tige), on écarte le crochet supérieur du crochet inférieur. Le crochet supérieur franchit donc successivement les crans de la portion crantée de la tige et, lorsque le redressement désiré est obtenu, on insère un dispositif de blocage (clip par exemple) entre le crochet supérieur et le cran le plus proche.

Par ailleurs, le système de compression comporte des crochets transversaires traversés par une tige filetée pourvue d'écrous. Les crochets supérieurs s'accrochent sur les apophyses transverses des vertèbres de la courbure proches de la vertèbre supérieure, tandis que les crochets inférieurs prennent appui sous les apophyses transverses ou la lame des vertèbres de la courbure proches de la vertèbre extrême inférieure. Un écrou est associé à chaque crochet et, par action sur lesdits écrous, on obtient la compression souhaitée.

Après mise en place de l'instrumentation, on réalise une arthrodèse et le patient est maintenu dans un corset pendant une durée comprise entre 6 et 12 mois.

Une telle instrumentation connue, quoique largement mise en œuvre, présente un certain nombre d'inconvénients, à savoir :

a) le réglage de l'ouverture de la courbure ne peut se faire de façon continue, mais au contraire par pas, chaque pas de réglage étant constitué par un cran de la portion crantée du système d'élongation. Par suite, le réglage définitif de l'ouverture du rachis est obtenu, non pas de façon exacte, mais à un cran près ;

b) l'ouverture de la courbure est obtenue par appui très localisé au niveau des deux seules vertèbres extrêmes de la courbure, de sorte que la pression exercée sur celles-ci est importante ;

c) les crochets, du système d'élongation notamment, peuvent tourner autour de la tige métallique de ce système, ce qui autorise des déplacements par rapport à leur mise en place initiale ;

d) aucune action directe de recentrage vers l'axe du tronc n'est exercée par l'instrumentation ;

e) aucune action réelle de dérotation n'est exercée sur les vertèbres du sommet ;

f) malgré une contention postopératoire prolongée, la rupture de la tige du système d'élongation, au raccordement des parties lisse et crantée de celle-ci, se produit dans 2,5 % des cas.

La présente invention a pour objet de remédier à ces inconvénients. Plus particulièrement, elle vise à obtenir une instrumentation suffisamment solide pour rendre superflue la longue contention postopératoire en plâtre ou corset, pendant le temps de la synostose vertébrale.

A ces fins, selon l'invention, le dispositif pour l'étaiement du rachis, comportant au moins une tige de section constante sur toute sa longueur et au moins deux organes d'ancrage pouvant prendre appui sur des vertèbres dudit rachis et pourvus de moyens de pression permettant de fixer lesdits organes d'ancrage en position sur ladite tige, lesdits moyens de pression coopérant avec la surface de ladite tige de sorte que lesdits organes d'ancrage peuvent être fixés, à la fois, en toute position longitudinale désiré le long de ladite tige et en toute position angulaire désirée autour de celle-ci, est caractérisé en ce que ladite tige présente des aspérités de surface et en ce que lesdits moyens de pression coopèrent avec lesdites aspérités de ladite tige.

On remarquera qu'un dispositif du type décrit dans le préambule de la définition ci-dessus est connu par le document DE-A-3 032 237. Grâce auxdites aspérités de surface, l'invention permet de solidariser les organes d'ancrage de la tige, de sorte qu'aucune rotation ne soit possible après actionnement desdits moyens de pression. Elle permet en outre de multiplier à volonté les organes d'ancrage et de répartir ceux-ci de façon que chaque vertèbre de la courbure soit fixée dans la position de redressement souhaitée. Par ailleurs, la tige étant de section constante, elle ne présente plus de point faible et sa rupture est empêchée.

Selon une particularité importante de la présente invention, les aspérités de ladite tige favorisent, en cas d'arthrodèse, l'accrochage de l'os néoformé, de sorte que ladite tige est parfaitement solidarisée à la zone osseuse fusionnée.

La tige peut être moletée ou guillochée.

On remarquera de plus que le document FR-A-2 458 271 décrit un dispositif pour l'étaiement du rachis dont la tige est filetée.

Les figures du dessin annexé feront bien comprendre comment l'invention peut être réalisée. Sur ces figures, des références identiques désignent des éléments semblables.

La figure 1 illustre schématiquement, de dos, un patient atteint de scoliose.

La figure 2 illustre la rotation relative des vertèbres d'un rachis scoliotique.

La figure 3 montre une instrumentation connue pour la correction chirurgicale d'une scoliose.

Les figures 4 à 9 montrent l'instrumentation conforme à l'invention.

Les figures 10, 11 et 12 illustrent un processus de mise en œuvre de l'instrumentation conforme à l'invention.

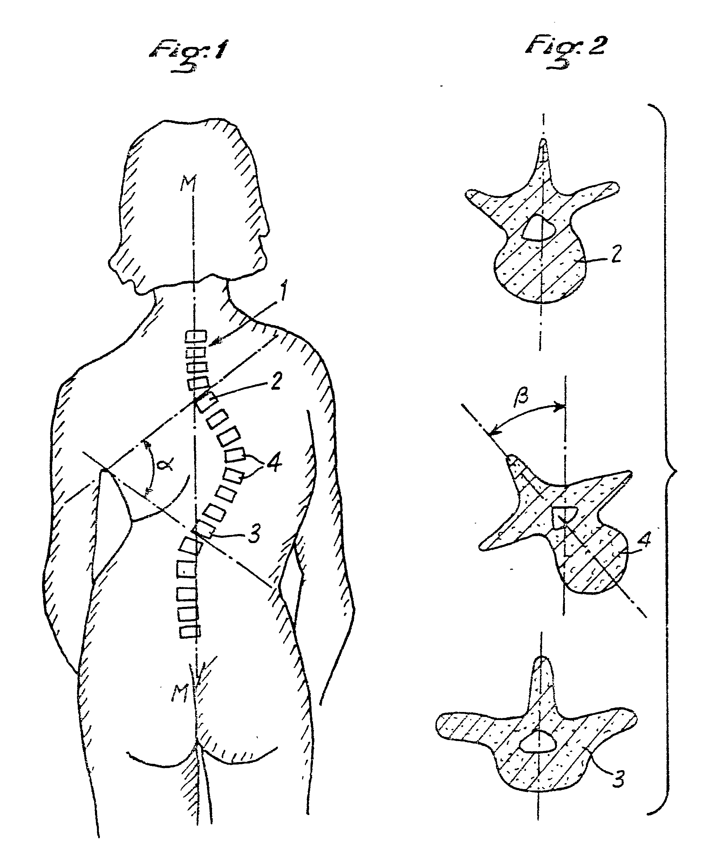

Sur la figure 1, on a représenté schématiquement de dos un patient atteint de scoliose et on a supposé que la colonne vertébrale 1 était visible. Les vertèbres sont schématisées par de petits rectangles ou trapèzes.

Ce patient présente une scoliose entraînant une déviation vertébrale vers la droite. La courbure scoliotique peut être définie grâce aux vertèbres extrêmes supérieure 2 et inférieure 3 de la déviation et à la ou aux vertèbres 4 se trouvant au sommet de la courbure. On remarquera que les vertèbres 2 et 3 sont celles qui sont les plus inclinées sur l'axe longitudinal médian MM du corps, tandis que les vertèbres 4 sont celles qui sont le plus éloignées de cet axe. L'angle $\alpha$ compris entre les plans des vertèbres 2 et 3 est donc une caractéristique de la courbure scoliotique. Comme on l'a mentionné ci-dessus, lorsque l'angle $\alpha$ dépasse un certain seuil (environ 50°), il est nécessaire d'avoir recours à l'arthrodèse et de mettre en place un étai du rachis.

Par ailleurs, comme illustré sur la figure 2, qui montre en vues de dessus schématiques chacune des vertèbres 2, 3 et 4, la vertèbre 4 de sommet de courbure subit une rotation sur elle-même d'amplitude $\beta$, du fait de la scoliose.

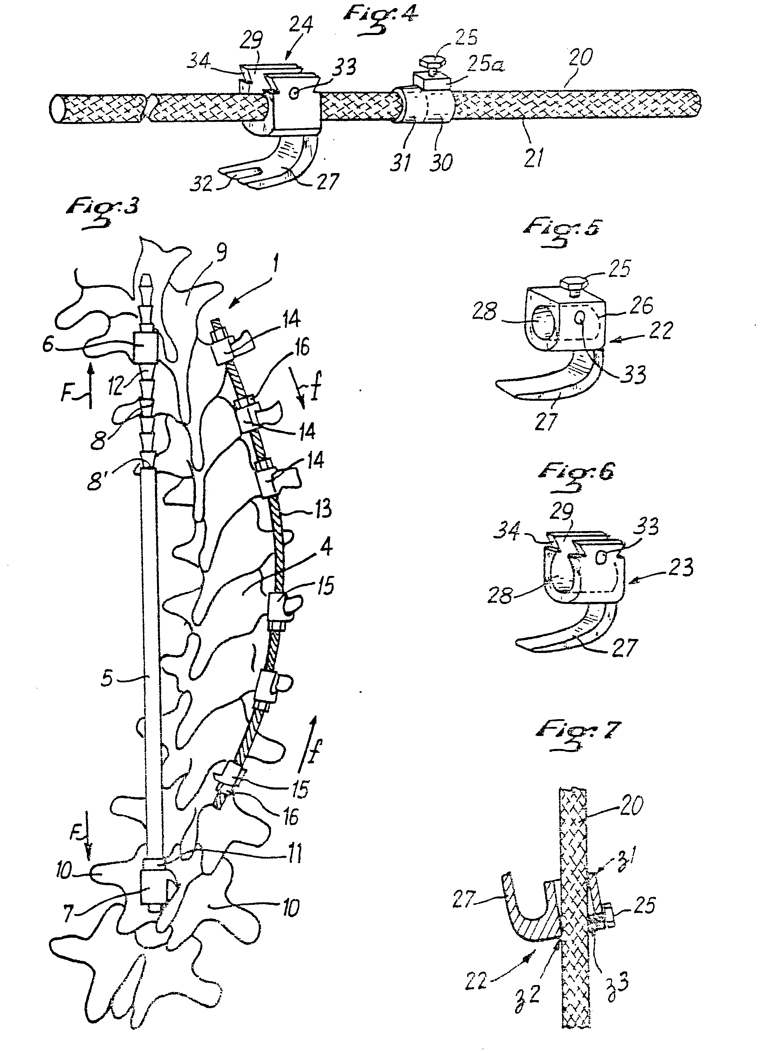

La figure 3 illustre la méthode et l'instrumentation connues de HARRINGTON pour redresser et étayer un rachis, et rappelées ci-dessus. Le système d'élongation comporte une tige métallique cylindrique 5 pouvant coopérer à ses deux extrémités avec des dispositifs d'ancrage 6 et 7. L'une des extrémités 8 de la tige 5 est crantée de façon à pouvoir régler l'écartement des dispositifs d'ancrage 6 et 7.

Généralement, l'organe d'ancrage supérieur 6, c'est-à-dire destiné à être fixé au côté de la partie supérieure du rachis, est accroché à une vertèbre dorsale 9 et son crochet (non visible sur la figure 3) est dirigé vers le haut et conformé de façon à pouvoir être inséré entre l'apophyse épineuse et une apophyse transverse de cette vertèbre, entre les facettes articulaires supérieure et inférieure, ce crochet pénétrant dans l'espace interarticulaire et prenant appui sur la vertèbre supérieure à ce niveau.

De même, l'organe d'ancrage inférieur 7, c'est-à-dire destiné à être fixé du côté de la partie inférieure du rachis, prend souvent appui sur une vertèbre lombaire 10. A cet effet, son crochet (peu visible sur la figure 3) est dirigé vers le bas et prend appui sur la lame vertébrale de cette vertèbre entre l'apophyse et le massif articulaire. Le coulissement de l'organe d'ancrage inférieur le long de la tige 5 est limité vers le haut par un épaulement 11 solidaire de cette dernière. Les vertèbres 9 et 10 de la figure 3 peuvent être les vertèbres extrêmes 2 et 3 de la figure 1.

Après mise en place des organes d'ancrage 6 et 7 sur leur vertèbre respective, on les écarte l'un de l'autre de la manière indiquée ci-dessus (flèches F), de façon à réduire la courbure scoliotique. Lorsque cette réduction est effectuée, on maintient l'écartement ainsi obtenu grâce à la mise en place d'un clip de blocage 12, disposé entre un cran de la partie 8 et l'organe d'ancrage 6.

La figure 3 fait clairement ressortir les inconvénients a, b, c, et f mentionnés ci-dessus en ce qui concerne le système d'élongation et notamment la possibilité de rupture de la tige 5 au raccord 8' de la partie crantée 8 avec le reste de la tige.

Par ailleurs, l'instrumentation de HARRINGTON peut comporter un système de compression comportant une tige filetée souple 13, sur laquelle coulissent trois organes d'ancrage supérieurs 14 et trois organes d'ancrage inférieurs 15. Ces organes d'ancrage 14 et 15 prennent appui chacun sur une vertèbre de la courbure du rachis 1 et sont pressés contre celles-ci par des écrous 16 se vissant sur la tige filetée 13, de sorte qu'ils exercent une action de compression (flèches f).

On voit ainsi que, même comportant un système de compression 13 à 16, l'instrumentation connue montrée par la figure 3 n'exerce aucune action directe de recentrage vers l'axe M-M et de dérotation des vertèbres 4 du sommet de la courbure (points d et e ci-dessus).

Pour remédier aux inconvénients a, c et f inhérents à cette instrumentation connue, la présente invention prévoit une tige d'étai 20 destinée à remplacer la tige 5. Comme on peut le voir sur la figure 4, la tige d'étai 20 selon l'invention a une section constante sur toute sa longueur (donc ne présente pas de·points faibles) et sa surface comporte une multitude d'aspérités 21, par exemple obtenues par moletage ou guillochage. Par ailleurs, on prévoit des organes d'ancrage 22, 23 ou 24 (voir les figures 4, 5 et 6) pouvant être fixés en position sur la tige 20, grâce à des vis de pression 25.

L'organe d'ancrage 22 (figure 5) comporte un corps 26 auquel est adjoint un crochet 27. Dans le corps 26 est percé un trou traversant 28, dont le diamètre est suffisant pour que ledit organe 22 puisse coulisser librement le long de la tige 20, lorsque celle-ci traverse le trou 28. Dans le corps 26, se visse une vis 25 dont l'extrémité débouche dans ledit trou 28. Ainsi, comme le montre la figure 7, l'organe d'encrage 22 peut être fixé en n'importe quelle position de la tige 20, par serrage de la vis 25, après avoir été enfilé sur ladite tige 20 par l'une quelconque des extrémités de celle-ci.

La mise en charge de l'organe 22 fait basculer celui-ci par rapport à la tige 20 (sur la figure 7 le basculement est exagéré à des fins illustratives) de sorte que le contact entre la tige 20 et ledit organe s'effectue par trois zones Z1, Z2 et Z3 espacées, lorsque la vis 25 est serrée. La fixation de l'organe d'ancrage 22 sur la tige 20 est donc parfaite, aussi bien en position longitudinale qu'en orientation autour de ladite tige, de sorte que ledit organe peut être utilisé aussi bien en traction qu'en compression, ainsi qu'en toute position de rotation.

Sur les figures 4 et 6, on a représenté des organes d'ancrage 23 et 24 à corps ouvert. En effet, puisque selon l'invention, les organes d'ancrage peuvent être fixés solidement en tous points de la tige 20, on peut les multiplier le long de celle-ci pour augmenter le nombre des prises le long du rachis ; il est donc intéressant de pouvoir mettre en place lesdits organes d'ancrage sur ladite tige, sans avoir à les rendre simultanément prisonniers de celle-ci, préalablement à la mise en place de la tige 20. Pour cela, les organes d'ancrage 23 et 24 comportent un canal 29 permettant l'introduction de la tige 20 et faisant déboucher le trou 28 à l'extérieur. Ainsi, les organes 23 et 24 peuvent à tout moment être mis en place sur la tige 20. A chacun des organes 23 ou 24 est associé un élément de blocage 30, portant une vis de pression 25 et enfilé sur la tige 20, avant mise en place de celle-ci. Chaque élément de blocage 30 présente la forme d'un manchon dont au moins une partie 31 est conique et est susceptible de coopérer avec une portion conique correspondante du trou 28 des organes 23 ou 24 (non visible sur les figures 4 et 6) pour bloquer lesdits organes sur la tige 20 par effet de coin. Il comporte de plus un corps 25a coopérant avec une partie correspondante de l'organe 23, 24, afin d'en bloquer la rotation autour de la tige 20.

Les organes 23 et 24 sont identiques, à ceci près que leur crochet 27 est légèrement différent. Le crochet 27 de l'élément 23, destiné à prendre appui derrière la lame d'une vertèbre (comme celui de l'organe 22) est plat et plein, tandis que le crochet 27 de l'élément 24, destiné à prendre appui sous le pédicule d'une vertèbre, comporte une fente 32 à cet effet.

On remarquera que pour leur mise en place, les organes d'ancrage comportent des trous 33 et/ou des découpes 34 en queue d'aronde servant de prise à des organes de préhension, tels qu'une pince.

Comme le montre la figure 8, les découpes en queue d'aronde 34 peuvent également servir à la solidarisation d'un élément 23 ou 24 avec la tige 20, notamment lorsque ledit élément doit subir de fortes contraintes. Dans ce cas, ·l'élément de blocage 30 comporte de plus des ailes 35 susceptibles de venir s'emboîter dans les découpes 34 en regard.

Pour renforcer encore la solidarisation d'un organe 23 ou 24 avec la tige 20, on peut prévoir un élément de blocage additionnel 36, constitué d'un anneau pourvu d'une vis de pression 25 et d'une saillie 37, susceptible de s'emboîter dans des découpes 34 disposées du côté opposé à l'élément de blocage 30.

Dans la variante de réalisation montrée par la figure 9, on prévoit un organe de blocage 39 comportant deux ailes latérales 40 susceptibles de venir enserrer les parois latérales des organes 23 et 24, afin d'éviter l'écartement éventuel de celles-ci. Par ailleurs, la face frontale desdits organes d'ancrage 23 et 24 vient en appui contre une face correspondante de l'organe de blocage 39, de sorte que tout basculement desdits organes 23 et 24 est empêché, dès serrage de la vis 25 de l'organe 39.

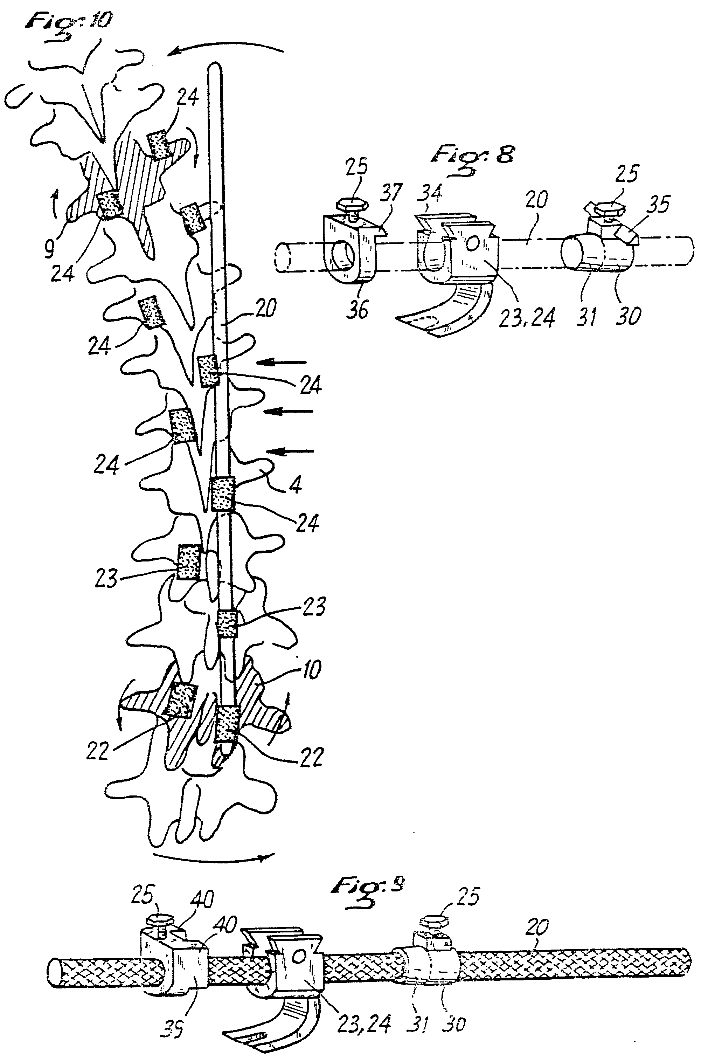

Les figures 10, 11 et 12 illustrent une possibilité de mise en œuvre du dispositif selon l'invention.

Au niveau de la vertèbre 10 inférieure, on commence par mettre en place deux organes d'ancrage 22, l'un inséré sur la lame du côté concave et l'autre sous la lame du côté convexe.

Ensuite, de bas en haut, on met en place de deux en deux vertèbres du côté concave et du côté convexe, soit des organes 23, soit des organes 24, de façon que lesdits organes disposés d'un côté soient décalés d'une vertèbre par rapport à ceux de l'autre côté (voir la figure 10). En prenant appui sur les organes 23 et 24 liés aux vertèbres de sommet 4, il est alors possible de procéder à la dérotation au moins partielle de celles-ci.

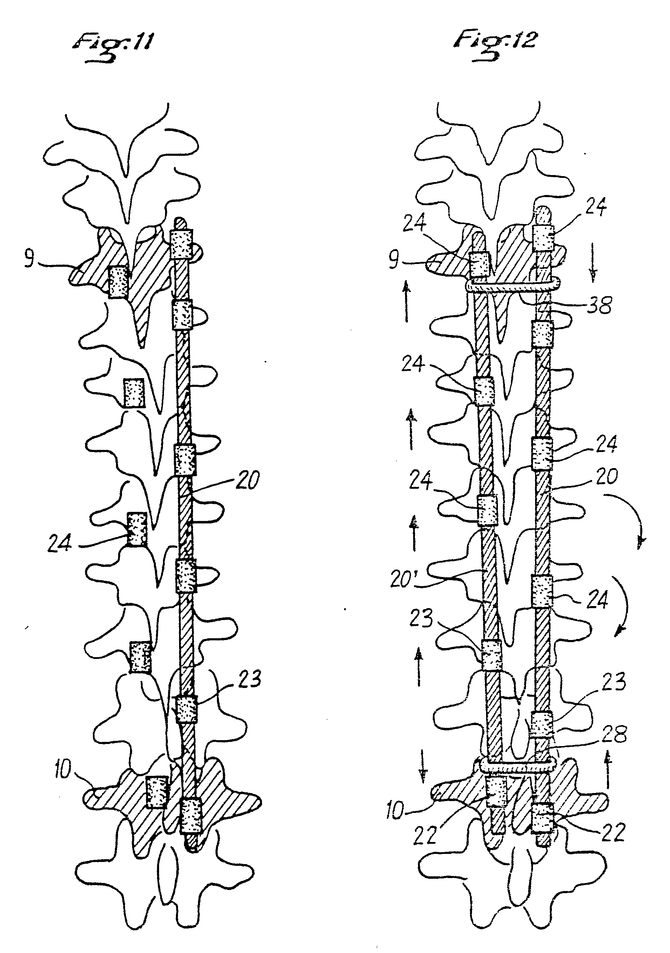

Puis on introduit une tige 20 dans l'organe inférieur 22, du côté convexe, et en se servant de cette tige comme levier, on introduit celle-ci successivement dans les organes 23 et 24 disposés au-dessus. On réduit donc par cette manœuvre la courbure dans le sens transversal, et on aligne au mieux les organes d'ancrage de la concavité (voir la figure 11).

On introduit alors une seconde tige, identique à la tige 20 et portant la référence 20' sur la figure 12, dans l'organe inférieur 22 de la concavité et, après avoir éventuellement cintré transversalement cette tige 20', on l'introduit successivement dans tous les organes 23 et 24 disposés dans la concavité de la courbure. Pour s'adapter aux courbures physiologiques antéropostérieures du rachis, les tiges peuvent être cintrées dans le plan sagittal sans risque de fragilisation du fait de la structure desdites tiges.

Bien entendu, les organes 23 et 24 des tiges 20 et 20' sont maintenus sur celles-ci grâce aux éléments de blocage 30 montés préalablement.

On remarquera que la mise en place des deux tiges 20 et 20' tend à produire la dérotation des vertèbres de sommet 4, par rapport aux vertèbres extrêmes 9 et 10.

On procède ensuite, pas à pas, à l'écartement vers le haut, à partir du milieu de la courbure du côté de la concavité, des organes 23 et 24 de la tige 20' et au rapprochement des organes 22 et 24 de la tige 20, du côté de la convexité, après quoi tous ces organes sont fixés en position grâce aux vis 25.

Les deux tiges 20 et 20' sont ensuite solidarisées l'une de l'autre par des entretoises transversales de traction 38, par exemple du type de celles décrites par FR-A-2 244 446. On pratique ensuite l'arthrodèse de façon connue.

Bien entendu, le mode de mise en œuvre décrit ci-dessus en regard des figures 10, 11 et 12 n'est donné qu'à titre illustratif, d'autres montages pouvant être réalisés suivant chaque cas particulier.

## Revendications

1. Dispositif pour l'étaiement du rachis (1), comportant au moins une tige (20, 20') de section constante sur toute sa longueur et au moins deux organes d'ancrage (22, 23, 24) pouvant prendre appui sur des vertèbres (2, 3, 4, 9, 10) dudit rachis (1) et pourvus de moyens de pression (25) permettant de fixer lesdits organes d'ancrage en position sur ladite tige (20, 20'), lesdits moyens de pression (25) coopérant avec la surface de ladite tige (20, 20'), de sorte que lesdits organes d'ancrage (22, 23, 24) peuvent être fixés, à la fois, en toute position longitudinale désirée le long de ladite tige et en toute position angulaire désirée autour de celle-ci, caractérisé en ce que ladite tige (20, 20') présente des aspérités de surface (21) et en ce que lesdits moyens de pression (25) coopèrent avec lesdites aspérités (21) de ladite tige (20, 20').

2. Dispositif selon la revendication 1, caractérisé en ce que les aspérités de surface (21) de la tige (20) sont formées par moletage, guillochage ou par une opération analogue.

3. Dispositif selon l'une des revendications 1 ou 2, caractérisé en ce que les organes d'ancrage (22) comportent d'une part, un corps (26) percé d'un trou traversant (28) dans lequel passe librement la tige (20) et pourvu d'une vis de pression (25) et, d'autre part, un crochet (27) solidaire dudit corps (26).

4. Dispositif selon l'une des revendications 1 ou 2, caractérisé en ce que les organes d'ancrage (23, 24) d'une part, comportent un corps (26) percé d'un trou traversant (28) dans lequel passe librement la tige (20) et débouchant à l'extérieur par un canal (29) pouvant être traversé par celle-ci, et, d'autre part, sont chacun associés à un élément de blocage (30) coulissant sur ladite tige (20) sur laquelle il peut être bloqué en position au moyen d'une vis de pression (25), un organe d'ancrage (23, 24) pouvant être solidarisé de l'élément de blocage (30) associé par coincement.

5. Dispositif selon la revendication 4, caractérisé en ce que, à chaque organe d'ancrage (24) est associé un élément de blocage additionnel (36, 39), coulissant également sur la tige (20) sur laquelle il peut être bloqué en position au moyen d'une vis de pression (25), cet élément de blocage additionnel (36, 39) étant disposé du côté de l'organe d'ancrage (24) opposé à l'élément de blocage (30).

6. Dispositif selon l'une des revendications 4 ou 5, caractérisé en ce que l'élément de blocage (30) comporte en plus de ses moyens de coincement (31), des moyens (25a, 35) s'emboîtant dans des parties complémentaires dudit organe d'ancrage (24).

7. Dispositif selon l'une quelconque des revendications 1 à 6, dans lequel ladite tige (20) s'étend sur une pluralité de vertèbres du rachis (1), caractérisé en ce qu'il comporte une pluralité d'organes d'ancrage (22, 23, 24) montés sur ladite tige et répartis le long de celle-ci.

8. Dispositif selon la revendication 7, caractérisé en ce qu'il comporte deux tiges (20, 20') s'étendant sur une pluralité de vertèbres du rachis et chacune disposée d'un côté de celui-ci, en ce que, sur chaque tige, sont montés une pluralité d'organes d'ancrage (22, 23, 24), et en ce que des entretoises transversales de traction (38) sont agencées entre lesdites tiges (20 et 20').

## Claims

1. Device for supporting the rachis (1), comprising at least one pin (20, 20') of constant cross-section throughout its length and at least two anchoring members (22, 23, 24) designed to rest on vertebrae (2, 3, 4, 9, 10) of said rachis (1) and provided with pressure means (25) cooperating with the surface of said pin (20, 20'), so that said anchoring members (22, 23, 24) may be fixed both in any desired longitudinal position along said pin and any desired angular position around the latter, characterized in that said pin (20, 20') has a rough surface (21) and in that said pressure means (25) cooperate with said rough surface (21) of said pin (20, 20').

2. Device according to claim 1, characterized in that said rough surface (21) of the pin (20) is obtained by milling, turning or like operation.

3. Device according to one of claims 1 or 2, characterized in that said anchoring members (22) comprise, on the one hand, a body (26) provided with a through hole (28) through which said pin (20) is freely slidable and which is equipped with a pressure screw (25) and, on the other hand, a hook (27) integral with said body (26).

4. Device according to one of claims 1 or 2, characterized in that said anchoring members (23, 24), on the one hand, comprise a body (26) with a through hole (28) through which said pin (20) is freely slidable, said hole opening outside by a groove (29) which can be traversed by said pin, and on the other hand, are each associated to a locking element (30) slidable on said pin (20) on which it can be locked in position by means of a pressure screw (25), one anchoring member (23, 24) being able to be interlocked with the associated locking element (30) by wedging.

5. Device according to claim 4, characterized in that an additional locking element (36, 39) is associated to every anchoring member (24), which locking element is also slidable on the pin (20) on which it can be locked in position by means of a pressure screw (25), said additional locking element (36, 39) being placed on the side of the anchoring member (24) opposite the locking element (30).

6. Device according to one of claims 4 or 5, characterized in that said locking element (30) comprises in addition to its wedging means (31), other means (25a, 35) which are engageable in complementary parts of said anchoring member (24).

7. Device according to any one of claims 1 to 6, wherein said pin (20) extends over a plurality of vertebrae of the rachis (1), characterized in that a plurality of anchoring devices (22, 23, 24) are mounted on said pin and distributed along the latter.

8. Device according to claim 7, characterized in that said device comprises two pins (20, 20') which extend over a plurality of vertebrae of the rachis, each pin being placed on one side thereof, in that on each pin are mounted a plurality of anchoring members (22, 23, 24), and in that tractional cross-pieces (38) are fitted between said pins (20 and 20').

## Patentansprüche

1. Vorrichtung zur Abstützung der Wirbelsäule (1) mit wenigstens einem Schaft (20, 20') von über seiner gesamten Länge konstantem Querschnitt und wenigstens zwei Verankerungselementen (22, 23, 24), die sich auf Wirbeln (2, 3, 4, 9, 10) der Wirbelsäule (1) abstützen können und die mit Spannmitteln (25) versehen sind, die das Feststellen der Verankerungselemente auf dem Schaft (20, 20') erlauben, wobei die Spannmittel (25) mit der Oberfläche des besagten Schaftes (20, 20') zusammenwirken, derart, dass die Verankerungsorgane (22, 23, 24) sowohl in jeglicher gewünschten Längsposition entlang dem Schaft als auch in jeglicher gewünschten Winkelposition gegenüber dem Schaft festgestellt werden können, dadurch gekennzeichnet, dass der Schaft (20, 20') Oberflächenunebenheiten (21) aufweist und dass die Spannmittel (25) mit diesen Unebenheiten (21) des Schafts (20, 20') zusammenwirken.

2. Vorrichtung gemäss Anspruch 1, dadurch gekennzeichnet, dass die Oberflächenunebenheiten (21) des Schafts (20) durch Rändeln, Guillochieren oder einen analogen Vorgang gebildet werden.

3. Vorrichtung gemäss Anspruch 1 oder 2, dadurch gekennzeichnet, dass die Verankerungselemente (22) einerseits einen Körper (26), durchbohrt mit einem Durchgangsloch (28), durch das der Schaft 20 sich frei bewegen kann, und versehen mit einer Spannschraube (25), und andererseits einen fest mit dem Körper (26) verbundenen Haken (27) umfassen.

4. Vorrichtung gemäss Anspruch 1 oder 2, dadurch gekennzeichnet, dass die Verankerungselemente (23, 24) einerseits einen Körper (26), durchbohrt mit einem Durchgangsloch (28), durch das der Schaft (20) sich frei bewegen kann und das sich nach aussen über einen Kanal (29) öffnet, durch den der Schaft (20) geführt werden kann, umfassen, und dass sie andererseits einem auf dem Schaft (20) verschiebbaren Blockierelement (30) zugeordnet sind, das auf diesem mit einer Spannschraube (25) in Position blockiert werden kann, wobei ein Verankerungselement (23, 24) mit dem Blockierelement (30) durch Klemmung fest verbunden werden kann.

5. Vorrichtung gemäß Anspruch 4, dadurch gekennzeichnet, daß jedem Verankerungselement (24) ein zusätzliches Blockierelement (36, 39) zugeordnet ist, das ebenfalls auf dem Schaft (20) verschiebbar ist und auf diesem mittels einer Spannschraube (25) in Position blockiert werden kann, wobei dieses zusätzliche Blockierelement (36, 39) neben dem Verankerungselement (24) entgegengesetzt zum Blockierelement (30) angeordnet ist.

6. Vorrichtung gemäß Anspruch 4 oder 5, dadurch gekennzeichnet, daß das Blockierelement (30) zusätzlich zu seinen Klemmmitteln (31) Mittel (25a, 35) umfaßt, die in entsprechende Teile des Verankerungselements (24) hineinpassen.

7. Vorrichtung gemäß einem der Ansprüche 1 bis 6, wobei sich der Schaft (20) über eine Vielzahl von Wirbeln der Wirbelsäule (1) erstreckt, dadurch gekennzeichnet, daß sie eine Vielzahl von Verankerungselementen (22, 23, 24) umfaßt, die auf dem Schaft (20) angebracht und auf seiner Länge verteilt sind.

8. Vorrichtung gemäß Anspruch 7, dadurch gekennzeichnet, daß sie zwei Schäfte (20, 20') umfaßt, die sich über eine Vielzahl von Wirbeln der Wirbelsäule erstrecken und die auf deren beiden Seiten angeordnet sind, daß auf jedem Schaft eine Vielzahl von Verankerungselementen (22, 23, 24) angeordnet sind, und daß in Querrichtung Zwischenstücke (38) zwischen den Schäften (20 und 20') angeordnet sind.

0 128 058

Fig. 1

Fig. 2

1

0 128 058

*Fig:4*

*Fig:3*

*Fig:5*

*Fig:6*

*Fig:7*

Fig. 10

Fig. 8

Fig. 9

3

Fig:11

Fig:12